Europäisches Patentamt

European Patent Office

Office européen des brevets

(19)

(11) Numéro de publication: **0 396 434**
**A1**

(12)

# DEMANDE DE BREVET EUROPEEN

(21) Numéro de dépôt: 90400486.8

(51) Int. Cl.⁵: **A61K 39/395, A61K 47/00**

(22) Date de dépôt: **21.02.90**

(30) Priorité: **23.02.89 FR 8902338**

(43) Date de publication de la demande:
**07.11.90 Bulletin 90/45**

(84) Etats contractants désignés:
**GR**

(71) Demandeur: **IDEON CORPORATION**
**301 Penobscot Drive**
**Redwood City, CA 94063(US)**

(72) Inventeur: **Stanislawski, Marc**
**106 rue Champollion**
**F-94400 Vitry Sur Seine(FR)**
Inventeur: **Nedwin, Glenn**
**29 Fairway Place**
**Half Moon Bay, California 94019(US)**

(74) Mandataire: **Ores, Irène et al**
**CABINET ORES 6, Avenue de Messine**
**F-75008 Paris(FR)**

(54) **Compositions à propriétés cytolytiques specifiques vis-à-vis de cellules cibles appropriées et leurs applications.**

(57) Compositions à propriétés cytolytiques spécifiques vis-à-vis de cellules cibles appropriées.
Lesdites compositions comprennent en combinaison :
- un inhibiteur de la glutathion réductase ;
- au moins une immunotoxine comprenant une enzyme conjuguée à un ligand spécifique d'un antigène de surface desdites cellules cibles.
Applications de ces compositions en tant que médicaments anticancéreux et en tant qu'agents d'épuration de la moelle osseuse.

EP 0 396 434 A1

## COMPOSITIONS A PROPRIETES CYTOLYTIQUES SPECIFIQUES VIS-A-VIS DE CELLULES CIBLES APPRO-PRIEES ET LEURS APPLICATIONS.

La présente invention est relative à des compositions à propriétés cytolytiques spécifiques vis-à-vis de cellules cibles appropriées, à leur application en tant que médicaments anticancéreux ainsi qu'à leur application à l'épuration de la moelle osseuse.

Depuis le développement des anticorps monoclonaux (Amc), l'un des buts de la biotechnologie a été la synthèse d'immunotoxines, ou "magic bullets", c'est-à-dire d'Amcs spécifiques d'un type de cellule (habituellement une cellule cancéreuse) couplés à un agent capable de détruire lesdites cellules. En général un Amc est préparé de manière à avoir une spécificité pour un antigène présent à la surface de la cellule cible, de telle sorte que l'Amc se liera à la cellule cible, et lorsqu'il sera incorporé à ladite cellule, il transportera son conjugué toxique à l'intérieur de cette dernière.

Le conjugué toxique est, la plupart du temps, du ricin, ou la sous-unité A du ricin. La sous-unité B se lie aux résidus galactose omniprésents à la surface des cellules, tandis que la sous-unité A inactive enzymatiquement les ribosomes. Il a été estimé qu'une seule molécule de ricin peut inactiver tous les ribosomes d'une cellule. La cellule envahie, incapable de produire de nouvelles protéines, meurt. Comme la sous-unité B est capable de se lier à presque n'importe quelle cellule, elle doit être désactivée ou exclue de toute thérapeutique.

Dans le cas d'étude *in vitro*, le ricin est habituellement utilisé globalement, la sous-unité B étant saturée avec du galactose pour empêcher les liaisons non-spécifiques.

Dans les études *in vivo*, la sous-unité B est habituellement retirée et un conjugué Amc-ricin A est utilisé.

Bien que la conjugaison du ricin à l'Amc augmente beaucoup la sélectivité de la cytotoxicité du ricin, sa toxicité résiduelle, due à des liaisons non spécifiques et à des liaisons avec les récepteurs Fc, est encore importante.

D'autres agents toxiques, quelquefois utilisés dans les immunotoxines comprennent des atomes radioactifs, la toxine diphtérique et des agents utilisés en chimiothérapie anticancéreuse. Ces immunotoxines présentent une toxicité résiduelle similaire à celle des immunotoxines comprenant du ricin, et ce, pour les mêmes raisons.

S.J. KLEBANOFF (J. Exp. Med., 1967, 126, 1063-1076), décrit une méthode pour détruire des bactéries *in vitro*, en utilisant la myéloperoxydase, des ions iodures et du peroxyde d'hydrogène. Le peroxyde d'hydrogène peut être remplacé par une combinaison de glucose et de glucose oxydase, qui, en fait, est plus efficace que le peroxyde d'hydrogène. L'effet bactéricide est apparemment dû à la formation de liaisons carbone-iode directement, plutôt qu'à la formation d'iode suivie d'une iodination.

KLEBANOFF mentionne également que l'iodure peut être remplacé par du bromure ou du chlorure. Les conditions de l'expérience demandent 0,01 $\mu$M d'$H_2O_2$ en présence de 0,1 $\mu$M d'$I^-$, ou 0,5 $\mu$M de glucose en présence de 0,5 $\mu$g de glucose oxydase et 0,1 $\mu$M d'$I^-$, à pH 5.

Cependant, il faut noter qu'un pH de 5 est inférieur au pH physiologique et que la concentration d'iodure dans le sérum normal est inférieur à environ 1 $\mu$g/100 ml (moins de 0,079 $\mu$M).

STARKIE (WO 87/03205) décrit une composition pour détruire des cellules ou un organisme, *in vivo*, comprenant un anticorps spécifique à la cellule ou à l'organisme à détruire, lié à une enzyme toxique. STARKIE cite notamment la glucose oxydase, qui en produisant du peroxyde d'hydrogène, détruit les cellules par oxydation des composés de la paroi cellulaire. Cependant, de nombreux types de cellules sont résistants au peroxyde d'hydrogène en raison de la présence d'agents antioxydants endogènes tels que les glutathions, de telles cellules étant réfractaires à une composition de ce type.

T.J. SULLLIVAN et al., Res. Commun. Chem. Path. Pharmacol., 1973, 6, 709-717, décrit une composition pour le traitement de la filaire du chien due à *Dirofilaria immitis*, comprenant de la glucose oxydase (GO) conjuguée à un anticorps anti-*Dirofilaria immitis*, suivi d'un traitement à la peroxydase de raifort et à la phénylènediamine. Le conjugué GO lié à la surface du parasite, produit du $H_2O_2$, qui est à son tour consommé par la peroxydase de raifort en convertissant la phénylène diamine en radicaux cytotoxiques.

SULLIVAN décrit également la radioiodination des parasites par l'utilisation de lactoperoxydase libre et d'iodure en combinaison avec le conjugué GO, mais il n'a pas mis en évidence d'effet cytotoxique.

R.B. LAL et al., J. Immunol. Meth., 1985, 79, 307-318 décrit une composition, pour éliminer sélectivement des sous-populations de lymphocytes, comprenant des conjugués enzymes-anticorps. LAL a sélectionné des enzymes libres pour leur effet cytolytique et a déterminé que la glucose oxydase et la phospholipase C (PLC) sont cytotoxiques vis-à-vis de sous-populations de lymphocytes, tandis que la xanthine oxydase, la phospholipase A2 et la phospholipase D sont sans effet, et que la choline oxydase est efficace en présence de 10 mM de choline.

LAL a ensuite conjugué la glucose oxydase et la phospholipase C à un anticorps monoclonal de souris anti-IgG de rat. Ces conjugués se sont avérés sélectifs vis-à-vis de sous-populations de lymphocytes murins en présence de différents anticorps monoclonaux de rat antisouris, (spécifiques des antigènes de surface cellu laire). La GO ou la PLC ajoutées à $2,5.10^{-8}$ g/ml entraînent la mort de 25 % et 28 % de cellules, alors que la combinaison GO et PLC entraîne 75 % de mort cellulaire. La GO conjuguée ou la PLC conjuguée sont capables de détruire 85-90 % de thymocytes ciblés à $250.10^{-8}$ g/ml ; cependant, la GO conjuguée est cytotoxique vis-à-vis de cellules non-ciblées à $25.10^{-8}$ g/ml. De plus, au delà de 15 jours, l'activité du conjugué GO décline d'un facteur de 63 000, et a conduit LAL a arrêté ces tests.

J. PENE et al. Biochem. Internat., 1986, 13, 233-43 décrit la destruction de cellules de myélome *in vitro*. Cet article montre en effet, le potentiel tumoricide de conjugués enzyme-anticorps sur des cellules de myélome d'origine murine.

Cet article décrit en particulier des conjugués anticorps-glucose oxydase et anticorps-lactoperoxydase qui véhiculent ces enzymes, de manière spécifique au niveau de la membrane de cellules myélomateuses.

La toxicité de ces conjugués, vis-à-vis de cellules myélomateuses NSO est étudiée en fonction du pourcentage de libération de $^{51}$Cr. Les cellules myélomateuses peuvent être tuées *in vitro* par préincubation avec un antisérum de lapin spécifique de la tumeur, suivi d'une incubation avec de la glucose oxydase (GO) et de la lactoperoxydase (LPO) conjuguées à des anticorps monoclonaux Ig anti-lapin, suivi d'une incubation dans du glucose et du NaI. Le traitement utilisant tous les composés (avec NaI à 0,1 mM) est suffisant pour entraîner la libération de 85 % de $^{51}$Cr à partir des cellules myélomateuses *in vitro*, tandis que l'omission de l'un des composés précédemment défini entraîne une chute de la libération de $^{51}$Cr aux valeurs contrôle.

Le BCNU, également dénommé carmustine, est parfois administré dans le traitement de tumeurs du système nerveux central. En tant que moutarde azotée bifonctionnelle, il est considéré comme inhibant la DNA nucléotidyl transférase il interfère avec les acides nucléiques par alcoylation, et entraîne une série de troubles : altération de la structure de la guanine favorisant l'appariement avec la thymine ; clivage du cycle imidazole de la guanine ; formation de liaison entre deux restes guanines, réalisant des ponts entre les deux brins de DNA qui empêchent la replication ; dépurination du DNA provoquant une rupture du brin.

Sa forte solubilité dans les lipides lui permet de traverser la barrière hémato-encéphalique de telle sorte que sa concentration dans le liquide céphalo-rachidien est supérieure à sa concentration plasmatique.

Il présente, comme tous les anticancéreux de nombreux effets indésirables, notamment une dépression retardée des cellules de la moelle osseuse, une thrombocytopénie et une leucopénie durant 2 à 7 semaines ainsi qu'une hépatotoxicité dans 26 % des cas.

Le BCNU est rapidement éliminé de la circulation sanguine et a une demi-vie de 2 à 3 minutes.

Chez la souris, sa $DL_{50}$ est approximativement de 25 mg/kg, lorsqu'il est administré oralement, intrapéritonéalement et en sous cutanée.

Chez l'homme, il est administré en I.V., à 200 mg/m$^2$ de surface corporelle.

On a également montré que le BCNU inactive spécifiquement la glutathion réductase, qui joue un rôle important pour la protection cellulaire vis-à-vis des molécules productrices d'oxygène; un certain nombres d'Auteurs ont montré que la désactivation de la gluthation réductase peut être envisagée pour induire une cytotoxicité vis-à-vis des cellules tumorales.

J.R. BABSON et al., Biochem. Pharmacol., 1981, 30, 2299-2304, décrit des expérimentations *in vitro* montrant une cytotoxicité accrue de l'adriamycine sur les hépatocytes, lorsqu'elle est administrée avec du BCNU.

J.M. HARLAN et al., J. clin. Invest., 1984, 73, 706-713, montre que des cellules endothéliales traitées *in vitro* avec du BCNU puis par de la glucose oxydase sont plus sensibles aux dommages dus au peroxyde.

En effet, le cycle redox du glutathion joue un rôle dans le mécanisme de défense antioxydant.

Le BCNU inhibe sélectivement la glutathion réductase et un prétraitement de cellules endothéliales par du BCNU potentialise toujours la lyse liée à la présence d'$H_2O_2$.

La lyse de cellules prétraitées par du BCNU est également potentialisée par les complexes glucose-glucose oxydase et xanthine-xanthine oxydase.

B.E. STARKE et al., J. Biol. chem., 1985, 260, 86-92 ont montré que l'inhibition du système de la glutathion réductase par le BCNU rend les cellules plus sensibles à l'attaque du peroxyde.

Dans les expériences décrites, le peroxyde d'hydrogène est généré *in situ* en utilisant la glucose oxydase.

Il est en particulier montré que l'inhibition de la catalase potentialise la mort cellulaire par un mécanisme qui est insensible aux antioxydants, alors que l'inhibition du cycle de la glutathion réductase potentialise un mécanisme de mort cellulaire sensible aux antioxydants et accompagné de peroxydation lipidique et de production d'H$_2$O$_2$.

Cet article montre que des doses de glucose oxydase qui tuent moins de 20 % d'hépatocytes natifs, entraînent la mort de 50-70 % des cellules prétraitées avec du BCNU.

I.E. COTGREAVE et al., Biochem. Pharmacol., 1987, 36, 2899-2904, montrent que le BCNU potentialise la cytotoxicité du diquat (herbicide bipyridyle) qui produit de l'oxygène actif dans les cellules.

Toutefois, cet article étudie plus particulièrement l'inhibition de la cytotoxicité du diquat par l'Ebselen seul ou associé à de la glutathion réductase de la N-acétylcystéine, en utilisant comme modèle d'étude des hépatocytes traités avec du BCNU.

C.F. NATHAN et al., J. Exp. Med, 1981, 154, 1539-1553, décrit l'utilisation de glucose oxydase liée à des particules de latex pour traiter des tumeurs intrapéritonéales chez la souris.

La combinaison de glucose oxydase liée et de BCNU est synergique dans le traitement des tumeurs résistantes au peroxyde. Cependant, les particules sont séparées en grumeaux qui forment des dépôts solides, qui sont répartis dans la totalité de la cavité péritonéale.

M.G. BATTELLI et al. décrit un conjugué anti-corps-enzyme consistant en xanthine oxydase liée à un anticorps monoclonal. La xanthine oxydase catalyse l'oxydation de l'hypoxanthine en xanthine et celle-ci en acide urique.

La xanthine oxydase existe sous deux formes : la D forme et la forme O.

Lors de l'oxydation de l'hypoxanthine en xanthine, catalysée par la xanthine oxydase sous sa forme O, des radicaux libres cytotoxiques sont produits, ce qui aggrave les dommages cellulaires lors de la conversion de la forme D en la forme O.

Le conjugué décrit dans cet article est cytotoxique vis-à-vis des cellules cibles uniquement.

C.F. NATHAN et al., J. Exp. Med., 1980, 153, 766-782 montre que l'inhibition du cycle redox du gluthation des cellules tumorales augmente leur sensibilité vis-à-vis des macrophages inducteurs de cytolyse.

Le cycle redox glutathion participe à la défense des cellules tumorales contre les antioxydants et est inhibé par le BCNU.

Cet article montre également que les cellules tumorales prétraitées avec du BCNU sont plus sensibles à la lyse par le peroxyde.

P.K. NAKANE et al., J. Histochem. Cytochem., 1984, 32, 8, 894-898, montrent que le composant sécréteur (SC) et l'antigène carcinoembryonaire sont distribués différemment dans l'épithelium gastrointestinal normal et dans les cellules cancéreuses de l'épithelium gastroinstestinal.

L'hypothèse est que de l'anti CEA injecté en I.V. n'est pas accessible aux cellules normales mais formera des complexes avec les cellules cancéreuses.

Cet article décrit plus spécifiquement un conjugué efficace *in vivo* glucose oxydase-anti CEA injecté en I.V. puis suivi d'une injection de NaI.

G.W. PHILPOTT , Surg., 1973, 74, 1, 51-58, décrit un système *in vitro* conjugué anticorps-GO, associé à de la lactoperoxydase (LP), et des iodures (I⁻) et précise que le couplage d'enzymes puissantes à des anticorps anti-tumeur peuvent être utiles pour obtenir une thérapie antitumorale sélective.

Lorsque de l'iodure et une deuxième enzyme, la lactoperoxydase sont ajoutés au système, la cellule subit une iodination et meurt.

R.B. BASCH et al., J. Immunol. Methods, 1983, 56, 269-280, décrit d'autre part un conjugué anticorps-catalase qui rend les cellules résistantes au peroxyde d'hydrogène.

Les compositions de l'Art antérieur présentent cependant un certain nombre d'inconvénients :
- la plupart de ces compositions ont été testées uniquement *in vitro* et leur comportement *in vivo* n'est pas connu ;
- les compositions, qui ont été testées *in vivo*, ont l'inconvénient de présenter une toxicité non spécifique trop importante vis-à-vis de cellules pré sentes dans le même environnement que les cellules cibles ;
- les doses utilisées dans ces compositions sont toujours très toxiques pour l'Homme.

La Demanderesse s'est en conséquence donné pour but de pourvoir à des compositions qui répondent mieux aux nécessités de la pratique que les compositions de l'Art antérieur notamment en ce qu'elles présentent une spécificité accrue vis-à-vis de cellules cibles, notamment vis-à-vis des cellules immunes ou tumorales, tout en permettant l'administration de doses inférieures à celles administrées antérieurement.

La présente invention a pour objet une composition à propriétés cytolytiques spécifiques vis-à-vis de cellules cibles appropriées présentant au moins un antigène de surface, caractérisée en ce qu'elle comprend en combinaison :
- un inhibiteur de la glutathion réductase ;
- au moins une immunotoxine comprenant une enzyme conjuguée à un ligand spécifique d'un antigène de surface des cellules cibles.

Selon un mode de réalisation avantageux de ladite composition, celle-ci comprend en combinaison :
- un inhibiteur de la glutathion rédacteuse ;
- une première immunotoxine comprenant une enzyme $E_1$ productrice d'oxygène réactif conjuguée à un ligand A spécifique d'un antigène de surface desdites cellules cibles (immunotoxine $E_1$-A) ; et
- une deuxième immunotoxine comprenant une enzyme $E_2$, productrice de dérivés halogénés en présence d'un halogénure et de l'oxygène réactif produit en présence de l'enzyme $E_1$, conjuguée à un ligand B capable de se lier spécifiquement à un antigène de surface desdites cellules cibles (immunotoxine $E_2$-B).

Selon un mode de réalisation préféré de la composition conforme à l'invention, l'inhibiteur de la glutathion réductase est une nitrosourée.

Selon une disposition de ce mode de réalisation, la nitrosourée est avantageusement la 1-3 bis (2-chloroéthyl)-1-nitrosourée, également dénommée carmustine ou BCNU.

Selon un autre mode de réalisation avantageux de l'invention, l'enzyme conjuguée à un ligand pour former une immunotoxine ou l'enzyme $E_1$ de la première immunotoxine susdite, est choisie dans le groupe qui comprend la glucose oxydase, la NADPH oxydase, la xanthine oxydase, la cholestérol oxydase, la choline oxydase, l'acide lactique oxydase et la pyruvate oxydase.

Dans le cas où la composition à propriétés cytolytiques comprend en combinaison un inhibiteur de la glutathion réductase et une seule immunotoxine, cette dernière est avantageusement constituée par un conjugué d'une enzyme $E_1$ productrice d'oxygène réactif et d'un ligand A spécifique d'un antigène de surface des cellules cibles.

De préférence, l'enzyme générant de l'oxygène actif ($E_1$) utilise un substrat présent chez l'homme en quantités suffisantes pour que ladite enzyme $E_1$ génère l'oxygène actif en quantité suffisante pour obtenir une cytotoxicité convenable.

Par exemple la GO (glucose oxydase) génère suffisamment d'$H_2O_2$ en présence du glucose présent dans le sérum pour détruire les cellules cibles choisies.

Ladite enzyme $E_1$ peut être d'origine humaine ou d'origine animale. Des enzymes recombinantes incluant des fragments d'enzymes comprenant le site actif peuvent également être utilisées.

Selon un autre mode de réalisation de la composition conforme à l'invention, l'enzyme $E_2$ de la deuxième immunotoxine susdite, est choisie dans le groupe qui comprend la myéloperoxydase, la lactoperoxydase, l'éosinophile peroxydase et la peroxydase salivaire.

Le substrat de l'enzyme $E_2$ est un halogénure et est administré concomitamment à la composition conforme à l'invention. Ledit halogénure est administré séparément et est notamment du iodure de sodium, non toxique même à dose élevée.

Selon un autre mode de réalisation de la composition conforme à l'invention, le ligand conjugué à une enzyme pour constituer une immunotoxine est choisi dans le groupe qui comprend les antisérums, les anticorps polyclonaux, les anticorps monoclonaux et les fragments d'anticorps, capables de se lier spécifiquement à un antigène de surface de la cellule cible.

On entend par ligand, une substance capable de se lier à un antigène de surface désiré tout en ne se liant pas aux cellules non cibles.

Conformément à l'invention, dans le cas où la composition comprend les deux immunotoxines $E_1$-A et $E_2$-B définies plus haut, le ligand A et le ligand B peuvent être identiques ou différents et/ou l'autre, peuvent être avantageusement choisis parmi les ligands des récepteurs de l'antigène cellulaire T et les ligands de l'immunoglobuline de surface.

L'immunoglobuline de surface est un marqueur préféré des lymphomes.

On entend par fragments d'anticorps notamment les fragments Fab et les fragments F(ab′)$_2$.

Le terme conjugué signifie que l'enzyme et le ligand sont connectés par des liaisons covalentes. Ceci est accompli par des moyens connus en soi et généralement appliqués aux anticorps marqués avec des enzymes pour les tests ELISA, typiquement en utilisant des réactifs de liaison bifonctionnelle tels que le SPDP, le MBS et le SMCC et analogues.

Chaque molécule de ligand peut être liée à une ou plusieurs molécules d'enzyme. L'enzyme et le ligand peuvent également être conjugués à l'aide d'une méthode recombinante, c'est-à-dire par l'expression de l'enzyme et du ligand comme une seule protéine de fusion. Par exemple, dans le cas des thymocytes commes cellules cibles, et avec comme antigène de surface le récepteur de l'interleukine-2, on peut préparer un vecteur d'expression (un plasmide) porteur du gène codant pour l'IL-2 (ou sa fraction de liaison) et fusionné avec un gène codant pour la glucose oxydase.

L'expression de ce gène composé produit une proteine de fusion IL-2/GO, ayant un domaine de liaison à l'IL-2 capable de se lier au récepteur de l'IL-2 de la cellule T, et un domaine GO capable d'une action cytotoxique.

Conformément à l'invention, les cellules cibles appropriées sont choisies dans le groupe qui comprend les cellules tumorales, les bactéries, les champignons, les protozoaires, les cellules autoimmunes, les cellules inflammatoires, les cellules infectées par un virus, les levures, les virus, les parasites.

On entend par cellule cible, n'importe quelle cellule que l'on désire détruire et qui comporte au moins un antigène de surface qui permet de la distinguer des cellules non-cibles.

On entend par antigène de surface, une protéine, un glucide, un lipide qui est caractéristique de la cellule cible et qui est capable de lier spécifiquement un ligand approprié.

Une telle composition présente un certain nombre d'avantages inattendus par rapport aux compositions de l'Art antérieur en effet, non seulement il existe un effet cytolytique synergique entre la nitrosourée et l'immunotoxine $E_1$-A et entre la nitrosourée et les immunotoxines $E_1$-A et $E_2$-B, mais, les doses de nitrosou rées sont nettement diminuées et l'on obtient de plus un gain de spécificité, dû à la présence d'au moins l'une de ces immunotoxines.

En outre, la présence de la deuxième immunotoxine ($E_2$-B) neutralise, de manière inattendue, la toxicité non spécifique de la première immunotoxine ($E_1$-A) et présente, de ce fait, à la fois une synergie de la toxicité spécifique et une neutralisation de la toxicité non spécifique.

Selon encore un autre mode de réalisation, la composition conforme à l'invention contient en outre, une autre immunotoxine qui comprend une catalase conjuguée à un ligand C approprié, capable de se lier aux antigènes de surfaces de cellules présentes dans le même milieu que les cellules cibles (immunotoxine catalase-C) et qui est associée soit à la composition comprenant un inhibiteur de la glutathion réductase et une immunotoxine $E_1$-A, soit à la composition comprenant un inhibiteur de la glutathion réductase et les immunotoxines $E_1$-A et $E_2$-B.

Une telle composition conforme à l'invention permet d'accroître encore la spécificité vis-à-vis des cellules cibles, en accroissant la protection vis-à-vis des cellules que l'on ne désire pas détruire.

La catalase présente des propriétés protectrices à partir de 2 $\mu$g/ml.

La composition conforme à l'invention est avantageusement mise sous une forme qui permet, en l'associant à un véhicule approprié, son administration de préférence par voie parentérale et avantageusement en IV ou en perfusion.

Comme tous les médicaments utilisés en chimiothérapie, il est difficile de définir les doses à administrer, celles-ci étant adaptées au patient et au type de tumeur.

La quantité efficace de BCNU, bien que celui-ci soit un agent thérapeutique connu et que son index thérapeutique ait déjà été établi, varie en fonction des cellules cibles, de leur dépendance vis-à-vis de la gluthation réductase et de leur sensibilité à la lyse par le peroxyde.

Cependant, de manière générale, la quantité efficace de BCNU sera celle qui permettra d'obtenir une concentration comprise entre 0,03 et 10 $\mu$g/ml, de préférence entre environ 0,1 et 3 $\mu$g/ml, ceci correspondant à environ 0,15-50 mg/dose, de préférence 0,5-15 mg/dose, alors que la posologie usuelle est de 100 mg/dose.

La quantité efficace en immunotoxines est encore plus difficile à établir et dépend de beaucoup de paramètres.

En général, une quantité efficace de chacune des immunotoxines sera celle qui fournit une concentration comprise entre 0,5 et 10 $\mu$g/ml. Ceci correspond à environ 5-25 mg/dose.

La composition conforme à l'invention peut avantageusement être injectée en deux étapes successives : d'abord le BCNU, puis l'immunotoxine ou le mélange d'immunotoxines l'halogénure est ensuite avantageusement administré par voie orale.

Conformément à l'invention, ladite composition comprend, pour une dose, une quantité en BCNU comprise entre 0,15 et 50 mg et une quantité en immunotoxine $E_1$-A comprise entre 5 et 25 mg, ainsi que, si la deuxième immunotoxine $E_2$-B est présente, une quantité d'immunotoxine $E_2$-B comprise entre 5 et 25 mg.

Dans le cas des cancers, une composition cytotoxique conforme à l'invention permet l'élimination des cellules tumorales et permet notamment l'obtention d'une rémission de la maladie.

Dans le cas des maladies autoimmunes, une telle composition permet l'élimination spécifique des lymphocytes autoimmuns entrainant une réponse immunitaire contre certains organes propres à l'individu concerné, tel que les cellules T-cytotoxiques vis-à-vis des cellules des îlôts de Langerhans, dans le diabète insulino-dépendant.

La composition conforme à l'invention est également applicable aux maladies infectieuses (bactériennes, virales et parasitaires).

La présente invention a également pour objet un procédé d'épuration *ex vivo* de la moelle osseuse de cellules cibles indésirables présentant au moins un antigène de surface, caractérisé en ce qu'il comprend les étapes suivantes :

(a) aspiration des cellules de la moelle osseuse,

(b) mise en contact desdites cellules avec une composition conforme à l'invention, pendant un temps approprié.

La composition conforme à l'invention est constituée par la combinaison d'un inhibiteur de la glutathion réductase, soit avec une immunotoxine $E_1$-A comprenant une enzyme $E_1$ productrice d'oxygène réactif conjuguée à un ligand A spécifique d'un antigène de surface des cellules cibles, soit avec deux immunotoxines, à savoir l'immunotoxine $E_1$-A précitée et l'immunotoxine $E_2$-B définie plus haut, soit avec trois immunotoxines, à savoir l'immunotoxine $E_1$-A, l'immunotoxine $E_2$-B et l'immunotoxine catalase-C également définie plus haut.

Une variante de ce procédé comprend la mise en contact successive des différents composants de la compositions conforme à l'invention.

Ledit procédé comprend alors les étapes suivantes :

(a) aspiration des cellules de la moelle osseuse,

(b) mise en contact desdites cellules avec une quantité efficace d'inhibiteur de la glutathion réductase puis

(c) mise en contact desdites cellules avec une quantité efficace d'une immunotoxine constituée par un conjugué d'une enzyme avec un ligand capable de se lier spécifiquement à un antigène de surface spécifique des cellules cibles.

Une autre variante de ce procédé comprend les étapes successives suivantes :

(a) aspiration des cellules de la moelle osseuse,

(b) mise en contact desdites cellules avec une quantité efficace d'inhibiteur de la glutathion réductase puis

(c) mise en contact desdites cellules avec une quantité efficace d'une première immunotoxine $E_1$-A, comprenant une enzyme $E_1$ générant de l'oxygène réactif conjuguée à un ligand A capable de se lier spécifiquement à un antigène de surface spécifique des cellules cibles, associée à une deuxième immunotoxine $E_2$-B, comprenant une enzyme $E_2$ productrice de dérivés halogénés en présence d'un halogénure et de l'oxygène réactif produit en présence de l'enzyme $E_1$, conjuguée à un ligand B capable de se lier spécifiquement à un antigène de surface desdites cellules cibles.

Selon un mode de mise en oeuvre avantageux du procédé d'épuration ex vivo de la moelle osseuse, il comprend en outre :

(d) la mise en contact desdites cellules avec une troisième immunotoxine catalase-C, comprenant une catalase conjuguée à un ligand C approprié, capable de se lier aux antigènes de surface de cellules présentes dans le même milieu que les cellules-cibles.

Selon un autre mode de mise en oeuvre avantageux de la présente invention, l'inhibiteur de la glutathion réductase est une nitrosourée.

Selon une disposition avantageuse de ce mode de mise en oeuvre, la nitrosourée est avantageusement la 1-3 bis (2-chloroéthyl)-1-nitrosourée, également dénommée carmustine ou BCNU.

Selon un autre mode de mise en oeuvre de ce procédé conforme à l'invention, l'enzyme $E_1$ est choisie dans le groupe qui comprend la glucose oxydase, la NADPH oxydase, la xanthine oxydase, la cholestérol oxydase, la choline oxydase, l'acide lactique oxydase et la pyruvate oxydase.

Selon un autre mode de mise en oeuvre du procédé conforme à l'invention, l'enzyme $E_2$ est choisie dans le groupe qui comprend la myéloperoxydase, la lactoperoxydase, l'éosinophile peroxydase et la peroxydase salivaire.

Selon encore un autre mode de mise en oeuvre du procédé conforme à l'invention, le ligand conjugué à l'enzyme $E_1$ ou à chacune des enzymes $E_1$, $E_2$, catalase, est choisi dans le groupe qui comprend les antisérums, les anticorps, les fragments d'anticorps capables de se lier spécifiquement à un antigène de surface de la cellule cible.

Selon encore un autre mode de mise en oeuvre du procédé conforme à l'invention, les cellules cibles à détruire sont des thymocytes et l'antigène de surface est une protéine caractéristique des thymocytes.

Selon une disposition avantageuse de ce mode de mise en oeuvre, ledit antigène de surface est choisi dans le groupe qui comprend l'antigène $T_4$, l'antigène $T_8$ et l'antigène Thyl.

Selon un autre mode de mise en oeuvre du procédé conforme à l'invention, les cellules cibles à détruire sont des cellules B de lymphomes.

Le procédé d'épuration ex vivo de la moelle osseuse conforme à l'invention, est applicable aussi bien dans le cas des autogreffes (épuration de la moelle osseuse des cellules cancéreuses) que dans le cas des allogreffes (épuration de la moelle osseuse à greffer des lymphocytes normaux qu'elle contient pour prévenir une réaction du greffon contre l'hôte (G.V.H.)).

Outre les dispositions qui précèdent, l'invention comprend encore d'autres dispositions, qui ressortiront

7

de la description qui va suivre, qui se réfère à des exemples de compositions conformes à l'invention, à la mise en évidence de leurs propriétés pharmacologiques ainsi qu'à des exemples de mise en oeuvre du procédé d'épuration.

Il doit être bien entendu, toutefois, que ces exemples sont donnés uniquement à titre d'illustration de l'objet de l'invention, dont ils ne constituent en aucune manière une limitation.

**Exemple 1 : Préparation des immunotoxines comportant un anticorps anti-lapin de chèvre (g).**

1.a. Préparation de glucose oxydase conjuguée à un anticorps anti-lapin de chèvre (g-GO).

La glucose oxydase (GO) est obtenue à partir d'*Aspergillus niger*.

L'anticorps anti-lapin de chèvre (g) peut être obtenu par des techniques standards ou peut être acheté (Antibodies Inc. par exemple). Par exemple, l'anticorps acheté chez Antibodies Inc., est purifié par chromatographie d'affinité sur Sépharose 4B conjuguée avec des Ig de lapin normales.

La GO est conjuguée à l'anticorps purifié à l'aide de glutaraldéhyde, comme décrit dans l'Art antérieur (AVRAMEAS, Immunochem., 1969, 6,43-52).

Les conjugués obtenus sont repurifiés par chromatographie d'exclusion sur une colonne S-300 dans 10 mM NaOAc, 150 mM Nacl, pH 5,8, de manière à éliminer l'enzyme non liée.

On peut également purifier les conjugués obtenus par immunoaffinité, en utilisant une colonne conjuguée à un anticorps monoclonal anti-Fv ou en utilisant une colonne conjuguée à l'antigène.

Le conjugué g-GO peut être testé pour son activité enzymatique en mesurant l'oxydation de l'odianisidine, en présence de peroxydase de raifort ; la réaction est suivie au spectrophotomètre par la mesure de la densité optique à 460 nm. Une courbe standard utilisant de la GO purifiée donne à 460 nm, environ 0,42 DO/min/$\mu$g d'enzyme.

1.b. Conjugué lactoperoxydase-anticorps anti-lapin de chèvre (g-LPO).

La lactoperoxydase (LPO) de lait d'origine bovine est achetée chez Boehringer Mannheim.

L'anticorps est obtenu comme dans l'exemple 1.a.

Le conjugué g-LPO peut être testé par la mesure de l'oxydation du 2,2'-azinobis-(3-éthyl-benzthiazoline sulfonique acide) en présence d'$H_2O_2$.

Une courbe standard, obtenue à 435 nm donne environ une densité optique de 2,5/min/$\mu$g d'enzyme.

Les conjugués obtenus en 1.a et 1.b peuvent être conservés en solution, ou de préférence lyophilisés.

**Exemple 2 : Préparation d'immunotoxines comportant un anticorps DLC48.**

De telles immunotoxines ont comme cellules cibles spécifiques les cellules de lymphome SU4.

L'anticorps DLC48 est précipité avec 50 % de sulfate d'ammonium et désalé sur une colonne de Biogel P6.

Il est ensuite couplé à des enzymes (GO, LPO, MPO) en utilisant un procédé SPDP/2-iminothiolane (2-IT) décrit par EISEN modifié (quantités de 2-IT diminuées, pH plus bas).

On obtient ainsi les immunotoxines suivantes : DLC48-GO, DLC48-LPO, DLC48-MPO.

Les quantités en enzyme et en anticorps des conjugués sont calculées comme suit : on estime tout d'abord l'activité enzymatique d'un conjugué donné, puis l'anticorps est estimé par soustraction de la valeur de l'enzyme du conjugué total à 280 nm. Ceci donne une approximation de la quantité réelle en anticorps.

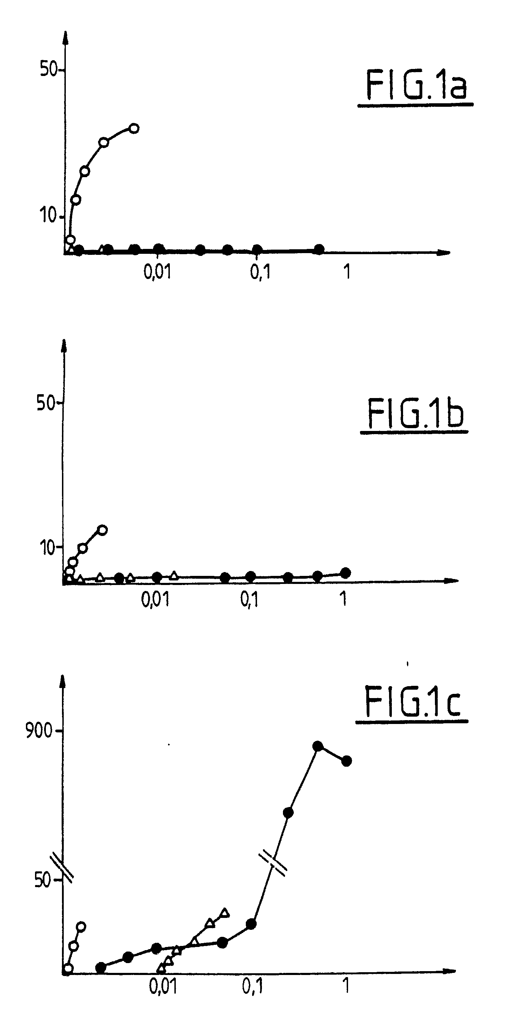

La figure 1 montre la spécificité de ces immunotoxines vis-à-vis des cellules SU4 par rapport à des immunotoxines contrôle comprenant comme anticorps, le MOPC104E.

$5.10^5$ cellules ($10.10^6$ cellules/ml dans une solution de Dulbecco équilibrée en sel, associée à du sérum bovin fétal à 2,5 %) sont incubées pendant 30 min à 37° C avec des quantités croissantes d'immunotoxines et les cellules présentant une activité enzymatique sont estimées par spectrophotométrie.

La figure 1a montre que les conjugués DLC48-G0 (O) se lient spécifiquement avec les cellules de lymphome SU4 et saturent $5.10^5$ cellules SU4 à approximativement 35 ng d'enzyme liée pour moins de 10 $\mu$g/ml de conjugué. Le conjugé contrôle MOPC104E-GO ($\Delta$) et la GO native ($\bullet$) ne présentent pas une liaison supérieure à la ligne de base.

La figure 1b montre des résultats semblables avec le conjugué LPO (O).

La saturation est, en ce cas, obtenue autour de 20 mg/5.10$^5$ cellules SU4.

Il n'y a pas de liaisons non spécifiques avec la MOPC104E-LPO (▲) ou la LPO native (●).

Les liaisons des conjugués MPO sont représentées sur la figure 1c. Le conjugué se lie aux cellules SU4 de la même manière que les autres conjugués.

## Exemple 3 : Préparation d'immunotoxines comportant comme anticorps un anticorps monoclonal spécifique des leucocytes humains (acm 097).

L'anticorps 097 est une IgM de souris qui présente une spécificité vis-à-vis des leucocytes humains et aucune réactivité vis-à-vis des cellules souches de la moelle osseuse.

Cet anticorps est plus particulièrement décrit dans Bone Marrow Transplant, 1987, 2 (suppl. 1), 158.

L'Acm 097 est purifié à partir d'ascite par précipitation séquentielle au sulfate d'ammonium et chromatographie, filtration sur gel sur Ultrogel Aca 34 équilibré avec un tampon Tris-HCl 0,5 M, pH 8,4 et KCl 0,1 M.

La glucose oxydase (GO) provient d'*Aspergillus niger*. L'activité enzymatique est de 242 U/mg. L'$E^{1\%}$ à 280 nm est de 13,6.

La lactoperoxydase (LPO) provient du lait d'origine bovine (220 U/mg, $A_{412}/A_{280}$ = 0,76). L'$E^{1\%}$ à 420 nm est de 13,9.

L'activité catalytique de la GO est mesurée au spectrophotomètre par oxydation de l'O-dianisidine en présence de peroxydase de raifort à 260 nm, et l'activité LPO est mesurée à 436 nm par l'oxydation de 2,2'-azinobis (3-éthylbenzthiazoline-6-sulfonique acide) (ABTS) en présence d'$H_2O_2$, comme spécifié dans l'exemple 1.

L'Acm 097 est couplé à la GO ou la LPO en utilisant le procédé de la N-succinimidyl-3-(2-pyridyl-dithio) propionate (SPDP) et le 2-iminothiolane, comme décrit dans l'exemple 1.

## Exemple 4 : Cytotoxicité *in vitro* de la composition conforme à l'invention.

4.a. Les cellules testées sont les lymphocytes humains du sang périphérique (LSP).

La GO et la LPO sont conjuguées à l'anticorps monoclonal spécifique des leucocytes humains 097, comme décrit dans l'exemple 3. Les LSPs sont purifiés à partir de sang humain par centrifugation en gradient de densité suivie de trois lavages (Ficoll) et placés dans un milieu DMEM (2.10$^5$/cellules par puits dans 50 μl) ou dans un milieu HepBS contenant du sérum de veau décomplémenté à 2 %. Les LSPs sont mises en suspension dans une composition conforme à l'invention. (BCNU : 0,003-30 μg/ml dans 0,09 % éthanol ; 097-GO : 0,15 μg/ml ; 097-LPO : 0,07 μg/ml).

Après incubation pendant 1/2 heure à 22°C, les cellules sont lavées deux fois et remises en suspension dans 50 μl de DMEM-HS 2,5 % contenant du glucose (4,5 g/l), puis on ajoute du NaI (0,1 mM final) pendant 10 min à 37°C.

Les cellules traitées sont alors mises en culture trois jours en présence de phytohémaglutinine (PHA), suivie d'un marquage de brève durée au $^3$H-TdR. L'incorporation de tritium est enregistrée et est reportée dans le Tableau I ci-après.

TABLEAU I

| Incorporation de $^3$H-TdR dans les cellules traitées (%) | | |
|---|---|---|
| BCNU (μg/ml) | Composition selon invention ou immunotoxines* | BCNU seul |
| 0 | 50, 60, 42 | 100 |
| 0,003 | 45 | 95 |
| 0,01 | 43 | 92 |
| 0,03 | 30 | 95 |
| 0,1 | 20 | 120 |
| 0,3 | 0 | 92 |
| 1,0 | 0, 0 | 82, 84 |
| 3,0 | 0 | 85, 68 |
| 10,0 | 0 | 27 |
| 30,0 | 0 | 3, 0, 17 |

\* immunotoxines seules

Le Tableau I montre que le BCNU seul n'entraîne pas une réduction significative des thymocytes à des concentrations inférieures à 10 μg/ml ; les immuno-toxines seules entraînent seulement 50 % de réduction en thymocytes.

D'autre part, des concentrations en BCNU comprises entre 0,1 et 3 μg/ml augmentent significativement la cytotoxicité des immunotoxines.

L'addition de quantités croissantes de BCNU entraîne une augmentation de la toxicité comme indiqué par la diminution d'incorporation de $^3$H-TdR.

Cet effet est nettement supérieur à l'effet additif des différents composants de la composition et montre bien l'effet synergique de la composition.

En utilisant un anticorps monoclonal spécifique d'antigènes caractéristiques de cellules tumorales appropriées, on peut traiter une leucémie ou un myélome sans dommage vis-à-vis des LSPs normaux.

Actuellement, le BCNU est habituellement administré à des doses de l'ordre de 100 mg/dose IV, ce qui correspond à une concentration sérique d'environ 100 μM (environ 20 μg/ml).

Comme le montre le Tableau I, cette concentration est toxique vis-à-vis des LSPs normaux. En utilisant le BCNU, en association avec les deux immunotoxines conformes à l'invention, on divise par environ 10, la quantité de BCNU à associer (0,5 à 15 mg/dose).

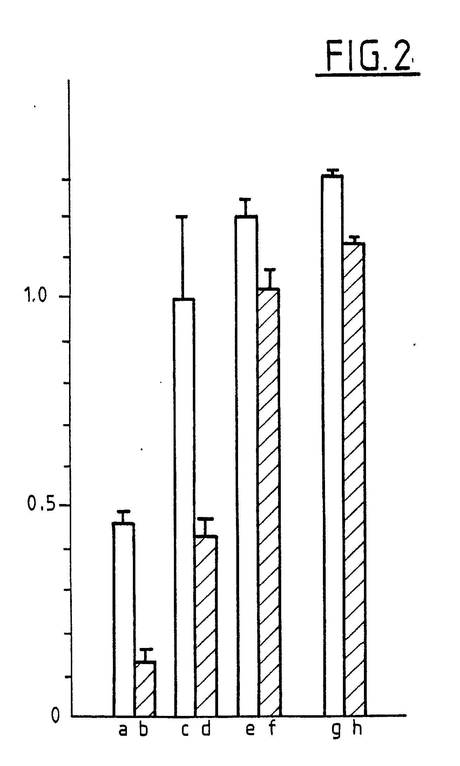

4.b. Les diagrammes de la figure 2 montrent le rôle du BCNU dans les compositions conformes à l'invention ; les conjugués utilisés sont le Ramb-GO et le Ramb-LPO.

Les anticorps Ramb sont obtenus chez le lapin et sont dirigés contre les membranes microsomiales de cellules du plasmocytome MOPC104E.

La figure 2 montre l'accroissement de cytotoxicité du au BCNU vis-à-vis de cellules ; en particulier, cette figure représente des histogrammes et montre l'absorbance à 540 nm, de différentes compositions testées. L'histogramme a montre l'absorbance correspondant à un mélange de Ramb-GO et de Ramb-LPO sans BCNU ; l'histogramme b montre l'absorbance correspondant à un mélange d'immunotoxines Ramb-GO et Ramb-LPO en présence de BCNU ; l'histogramme c montre l'absorbance correspondant à du BCNU seul ; l'histogramme d montre l'absorbance correspondant à l'immunotoxine Ramb-GO seule en présence de BCNU ; l'histogramme e montre l'absorbance correspondant à l'immunotoxine Ramb-LPO seule ; l'histogramme f montre l'absorbance correspondant à l'immunotoxine Ramb-LPO seule en présence de BCNU ; l'histogramme g est un témoin négatif (sans immunotoxine et sans BCNU) ; l'histogramme h est un témoin négatif (avec BCNU et sans immunotoxine).

Comme le montrent ces histogrammes, le BCNU augmente l'action aussi bien de l'immunotoxine Ramb-GO seule (histogramme c) que l'action d'un mélange Ramb-GO et Ramb-LPO (histogramme b).

**Exemple 5 : Rôle de la combinaison de deux immunotoxines dans la composition conforme à l'invention : effet synergique.**

L'effet des immunotoxines seules ou en combinaison sur la viabilité des cellules tumorales est étudié en utilisant trois tests différents : le test de libération $^{51}$Cr, le test colorimétrique MTT et la clonogénicité *in vitro*.

Ces tests permettent d'obtenir des réponses similaires avec les différentes immunotoxines décrites.

A titre d'exemple non limitatif, les exemples 5.a, 5.b et 5.c montrent l'effet sur la viabilité cellulaire des immunotoxines g-GO et g-LPO selon l'exemple 1, vis-à-vis des cellules NSO (cellules de plasmocytome murin).

5.a : test de libération $^{51}$Cr.

- Les cellules NSO sont cultivées *in vitro* dans du DMEM supplémenté avec 7 % de sérum de cheval décomplémenté.

- Les conjugués (g-GO et g-LPO) sont préparés conformément au protocole de l'exemple 1 :

- les anticorps Ramb sont obtenus chez le lapin et sont dirigés contre les membranes microsomiales de cellules de plasmocytome MOPC104E les conjugués Ramb-GO et Ramb-LPO sont obtenus comme décrit dans l'exemple 1.

- Test proprement dit : 1 à 5.10$^6$ cellules tumorales sont marquées avec 100 $\mu$Ci de $^{51}$ chromate de sodium dans du DMEM-HS 10 % pendant une heure à 37°C dans un incubateur à $CO_2$ humide. Environ 1 500 à 4 500 cpm sont incorporés /10$^4$ cellules. Les cellules sont lavées et incubées pendant 20 min à 37°C dans un sérum de lapin contenant des anticorps anti-membrane de plasmocytome (Ramb) dilué au 1/15, ou dans du sérum de lapin normal décomplémenté (RNS), dans un milieu à raison de 10.10$^6$ cellules/ml dans des tubes de Falcon n° 2 001, comprenant une solution saline équilibrée de Dulbecco tamponnée au bicarbonate et du HS à 2,5 % décomplémenté (BBSS-HS 2,5 %). Les cellules sont lavées et 50 $\mu$l (1.10$^5$ cellules) sont distribuées dans les puits d'une microplaque, puis on incorpore 50 $\mu$l de différentes dilutions des immunotoxines (g-GO mélangé avec g-LPO ou chaque immunotoxine seule), lesdites dilutions étant réalisées dans du BSS-HS 2,5 %. Après 20 min d'incubation à 22°C, suivie de deux lavages, les cellules sont mises en suspension dans 100 $\mu$l de milieu complet (CM) : BBSS-HS ou -FBS 2,5 % supplémenté avec 4 g/l de dextrose et 0,1 mM NaI, transférées dans des tubes Falcon n° 2054 et placées à 37°C dans un incubateur à $CO_2$ humide pour réaliser la réaction cytotoxique. Les tubes sont retirés à des moments différents, centrifugés et la radioactivité libérée est déterminée sur un échantillon de surnageant de 50 $\mu$l. Le pourcentage de $^{51}$Cr libéré est déterminé à partir de la formule suivante :

$$\frac{\text{coups/min ds surnag. (50 } \mu\text{l)} - \text{coups/min B.F.}^{(*)}}{\text{coups/min ds surnag. (cell. mises en susp. ds sol. HCl 4N)} - \text{coups/min B.F.}} \times 100$$

et correspond au pourcentage de coups/min relargués spécifiquement. (*) B.F. = bruit de fond.

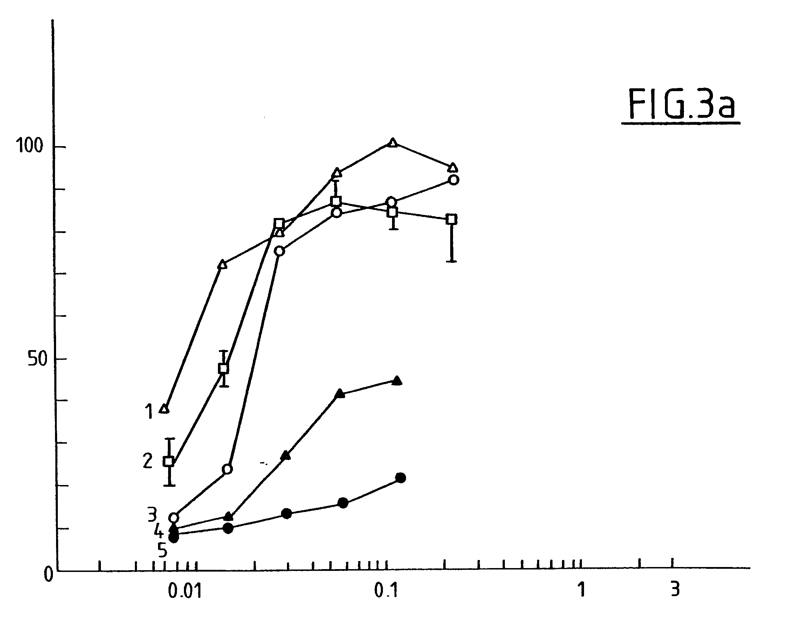

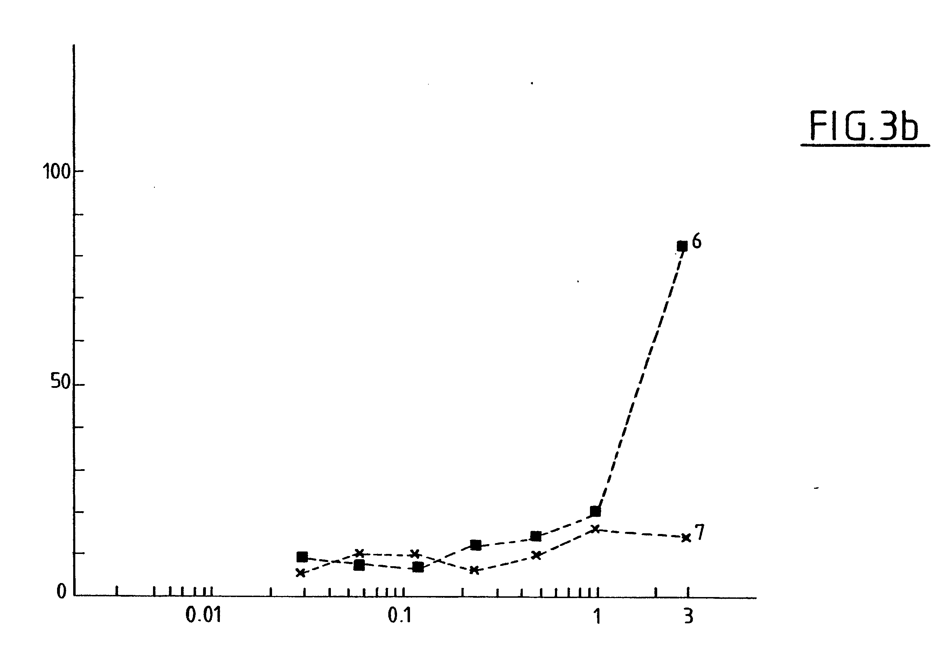

Les figures 3a et 3b montrent les quantités de $^{51}$Cr libérées à partir des cellules NSO lorsque ces cellules sont incubées comme précisé ci-dessus avec des doses différentes de g-GO et/ou de g-LPO.

La figure 3a comporte en abscisse la concentration en g-GO ajoutée et en ordonnée le pourcentage de $^{51}$Cr libéré. La courbe 1 correspond à une concentration en g-LPO de 0,48 $\mu$g/ml ; la courbe 2 correspond à une concentration en g-LPO de 0,12 $\mu$g/ml la courbe 3 correspond à une concentration g-LPO de 0,24 $\mu$g/ml ; la courbe 4 correspond à une concentration en g-LPO de 0,06 $\mu$g/ml ; la courbe 5 correspond à une concentration en g-LPO de 0,03 $\mu$g/ml.

La figure 3b montre que ni le g-LPO seul (courbe 6), ni le g-GO seul (courbe 7) ne provoquent une cytotoxicité à des doses où le mélange est cytotoxique ; seules des doses élevées de g-LPO seul (courbe 6) entraîne une cytotoxicité. On voit que la libération en $^{51}$Cr est maximale pour les mélanges de g-GO et g-LPO et lorsque ces mélanges contiennent des concentrations optimales en chacun des deux conjugués comme suit : 0,12 à 0,48 $\mu$g/ml de g-LPO et 0,03 à 0,25 $\mu$g/ml de g-GO.

5.b : essai MTT

11

Les cellules tumorales sont incubées dans des microplaques de microculture à une concentration de $1.10^6$/ml avec différentes dilutions de Ramb-GO et/ou de Ramb-LPO, lesdites dilutions étant réalisées dans du BSS-HS 2,5 % ou du BSS-HS 2,5 % tamponné Hepes (HBSS) ou du BSS-FBS 2,5 %. On utilise comme contrôle des cellules tumorales incubées avec des immunotoxines ne réagissant pas avec lesdites cellules. Après 20 min à 22° C, puis lavage, $1.10^5$ cellules sont transférées sur d'autres microplaques et mises en suspension dans 100 $\mu$l de CM et placées à 37° C dans un incubateur à $CO_2$ humide. Après 45 min à deux heures d'incubation à 37° C, les cellules sont lavées dans du DMEM-HS 2,5 %, mises en suspension dans 100 $\mu$l de DMEM-HS 2,5 % et 10 $\mu$l de colorant MTT tetrazolium (5 mg/ml) est ajouté à chaque puits. Après une nouvelle incubation de deux heures à 37° C, les microplaques sont centrifugées et 100 $\mu$l de DMSO sont ajoutés à chaque puits. La couleur développée est estimée spectrophotométriquement à 540 nm dans un appareil approprié. La densité optique des cellules tumorales non-traitée (100 % de viabilité) est comprise entre 0,8 et 1,4 de densité optique. Les résultats sont exprimés comme le pourcentage de l'absorbance-contrôle comme suit :

$$\frac{\text{DO des cellules traitées} - \text{DO de la ligne de base}}{\text{DO des cellules non-traitées} - \text{DO de la ligne de base}} \times 100$$

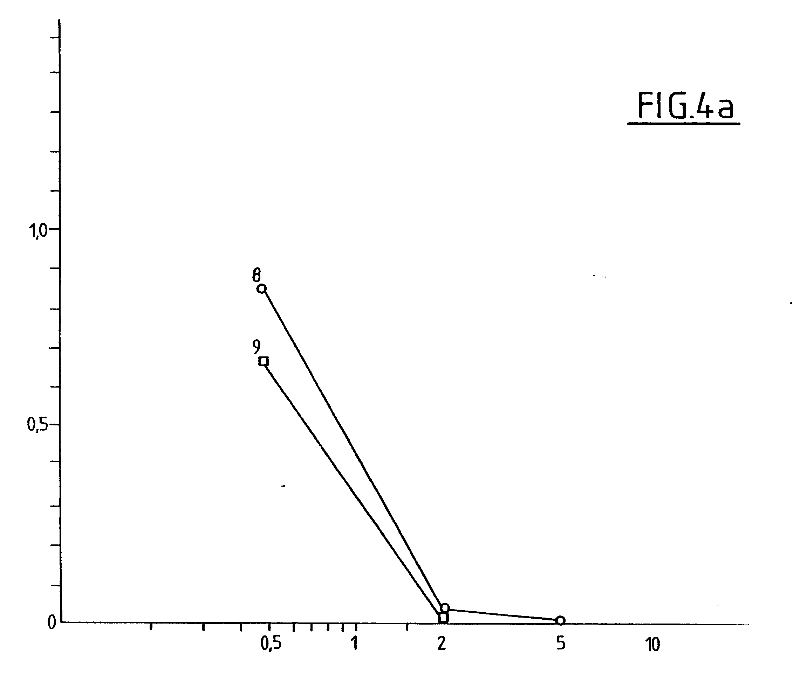

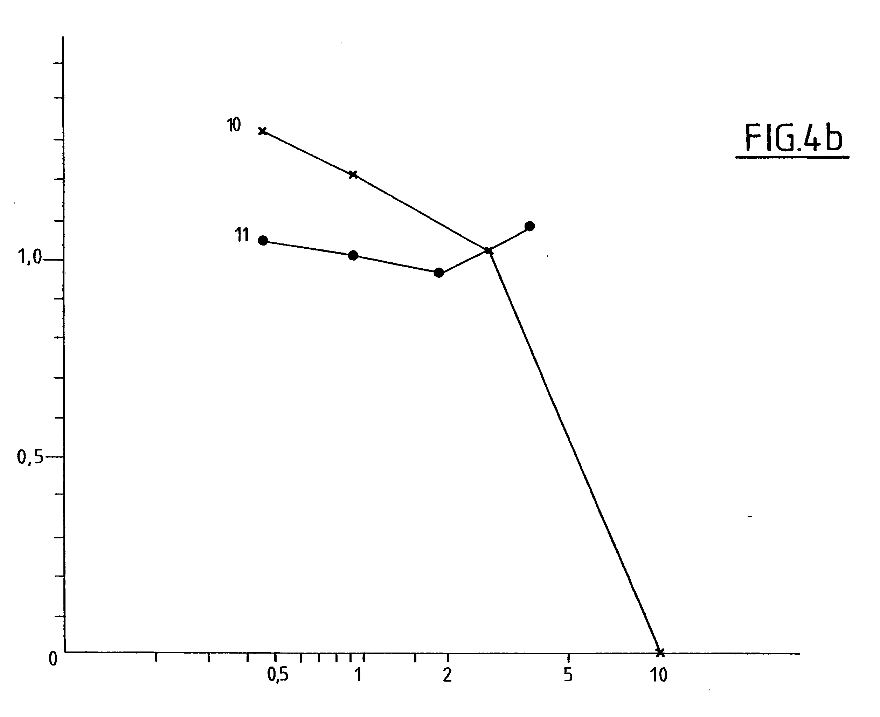

Les figures 4a et 4b montrent les résultats obtenus.

La figure 4a montre plus particulièrement les résultats obtenus avec un mélange des deux immunotoxines et comprend en abscisse la concentration en $\mu$g/ml de Ramb-LPO ajouté et en ordonnée la densité optique à 540 nm. La courbe 8 correspond à l'addition de Ramb-GO à une concentration de 1 $\mu$g/ml la courbe 9 correspond à l'addition de Ramb-GO à une concentration de 3 $\mu$g/ml. La mise en présence d'une dose aussi faible que 1 $\mu$g/ml de Ramb-GO associée à 2 $\mu$g/ml de Ramb-LPO réduit l'absorbance de plus de 99 %.

La figure 4b montre les résultats obtenus avec soit le Ramb-GO seul (courbe 10), soit le Ramb-LPO seul (courbe 11). Cette figure montre que lorsque les cellules NSO sont mises en présence de 3 ou 4 $\mu$g/ml, soit des conjugués Ramb-LPO seuls, soit des conjugués Ramb-GO seuls, ceux-ci ont peu d'effet sur la densité optique à 540 nm. Le conjugué Ramb-GO est toxique par lui-même seulement à des doses supérieures à 10 $\mu$g/ml. Les anticorps ne réagissant pas avec les cellules cibles n'entraînent aucune cytolyse, tel est le cas notamment de l'association Ramb-GO + Ramb-LPO vis-à-vis des cellules tumorales SU4. Une estimation de l'avantage, ou du gain quantitatif, de l'utilisation de la combinaison des deux immunotoxines peut être obtenue lorsqu'on calcule la cytotoxicité 50 % (environ une D.O. de 0,6) des courbes dose-réponse : le gain en cytotoxicité spécifique, obtenu avec l'association des deux immunotoxines est environ de 3. Ce calcul est effectué en comparant la courbe 8 (figure 4a) et la courbe 10 (figure 4b).

5.c : <u>essai de clonogénicité</u>

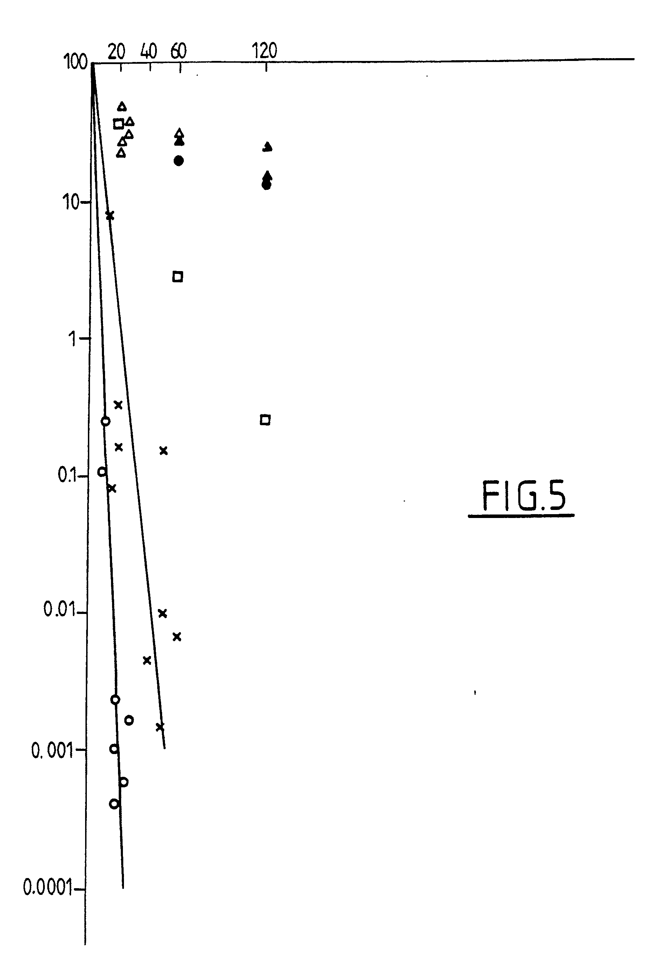

Les cellules tumorales sont incubées comme décrit dans l'exemple 4.a (test au $^{51}$Cr), la concentration cellulaire étant de $0,6.10^6$/ml. Après une incubation de 20 min à deux heures à 37° C dans du CM, les cellules sont lavées deux fois, mises en suspension dans du DMEM-HS 10 % et sont distribuées à des concentrations de $10^4$, $10^3$, $10^2$ et 10 cellules par puits dans des plaques de microculture à fond plat comprenant 96 puits. Les plaques sont cultivées à 37° C pendant 6 jours ; au sixième jour les cellules sont supplémentées avec 100 $\mu$l de milieu frais et au douzième jour de 100 $\mu$l de milieu est retiré et est remplacé par 100 $\mu$l de milieu frais. Les micro-plaques sont observées au microscope et les résultats sont exprimés par la fréquence de cellules survivantes. (figure 5)

Le nombre de cellules tumorales qui ne sont pas détruites est estimé au moyen du test de clonogénicité.

Tant des cellules NSO indirectement ciblées, c'est-à-dire tout d'abord incubées avec un sérum de lapin contenant des anticorps anti-membrane de plasmocytome puis incubées avec des conjugués g-GO et g-LPO, en association, que des cellules NSO directement ciblées avec Ramb-GO + Ramb-LPO sont détruites très efficacement.

La figure 5 comporte en abscisse le temps (en minutes) d'incubation dans le milieu CM, et en ordonnée, le pourcentage de cellules survivantes ; la destruction cellulaire est une fonction du temps : les cellules NSO sont ciblées indirectement par incubation en présence de Ramb suivie d'une seconde incubation avec l'association g-GO + g-LPO pour une concentration de 0,6 x $10^5$ cellules/ml (O) ou pour une concentration de 2,5 x $10^6$ cellules/ml (X), ou seulement avec g-GO pour une concentration de 0,6 x $10^6$ cellules/ml (●) ou avec g-LPO pour une concentration de 0,6 x $10^6$ cellules/ml (□).

Lorsque la première incubation est réalisée dans du sérum de lapin normal (NRS) à la place de Ramb, suivie d'une seconde incubation, on obtient la courbe suivante (g-GO + g-LPO pour 0,6 x $10^6$ cellules/ml (▲)). Le témoin négatif est représenté par la courbe (△), pour laquelle des cellules NSO (0,6 x $10^6$ cellules/ml) sont incubées avec du Ramb ou dans un milieu tel que DMEM-HS 10%.

Dans tous les cas, le g-GO est ajusté à 0,05 $\mu$g/ml et le g-LPO est ajusté à 0,3 $\mu$g/ml.

Après lavage, les cellules sont mises en suspension dans du CM, et aux temps indiqués sur l'axe des abscisses des échantillons sont retirés, lavés dans du DMEM-HS 10 % et mis en cultures sur des microplaques pour différentes concentration cellulaires. Les survivants sont comptés au 14ème jour.

Lorsque les conjugués sont utilisés seuls ((●) ; (□)), le g-GO (●) n'est pas cytotoxique et le g-LPO (□) est peu toxique (deux logs de décroissance de viabilité en environ 100 minutes) ; le couple g-GO + g-LPO (O ; X) est très toxique, le ciblage à 0,6 x $10^6$ cellules/ml étant optimal, entraîne un effet synergique des deux conjugués. En extrapolant la courbe, on peut obtenir six logs de décroissance cellulaire en un temps inférieur à 60 minutes, ce qui permet d'envisager l'épuration d'autogreffes.

Les témoins NSO ciblés avec du sérum de lapin normal (NRS) puis les deux conjugués (▲) et NSO sans adjonctions, ne sont pas cytotoxiques, ce qui démontre la spécificité du système.

**Exemple 6 : Spécificité de l'effet cytotoxique des immunotoxines conformes à l'invention.**

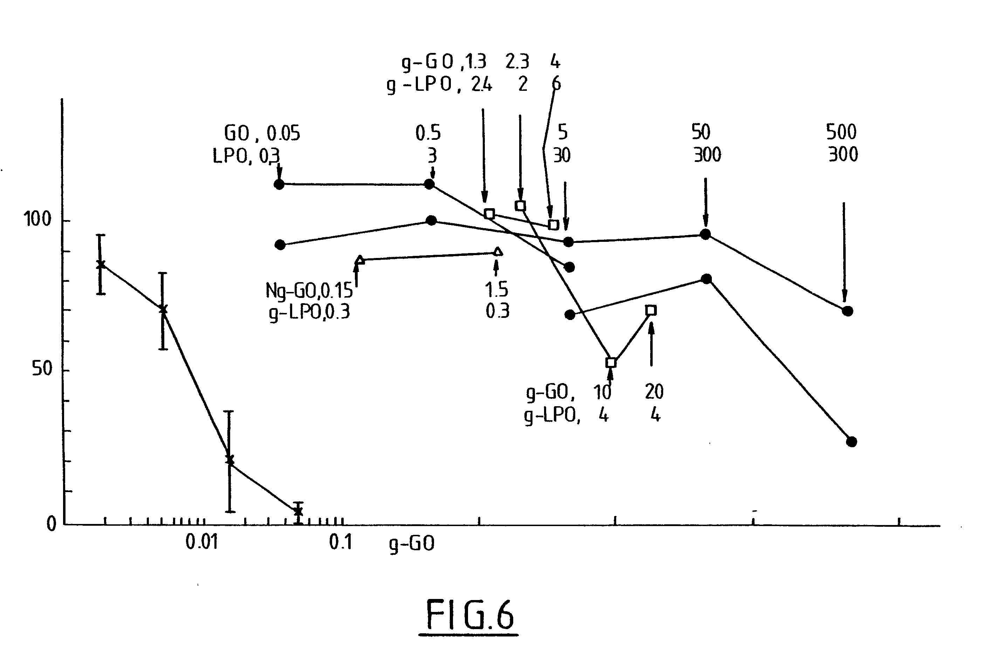

Ceci est réalisé avec un test MTT par estimation de la quantité de conjugués spécifiques versus la quantité de conjugués non spécifiques, ou les quantités d'enzymes natives GO ou LPO, ne réagissant pas avec les cellules testées. Comme le montre la figure 6 une quantité aussi faible que 0,01 $\mu$g/ml de g-GO mélangée à 0,3 $\mu$g/ml de g-LPO est suffisante pour obtenir un effet cytotoxique spécifique au point limite de 50 % et ceci est en accord avec les résultats obtenus avec le test de libération du $^{51}$Cr (figure 3a).

Par contre, l'incubation de cellules NSO avec des conjugués non spécifiques (ne réagissant pas avec lesdites cellules) ou des mélanges d'enzymes GO et LPO natives ne produisent qu'une cytotoxicité marginale. Le point limite de 50 % de cytotoxicité spécifique est séparé du point limite de cytotoxicité non spécifique par au moins 4 logs, ce qui correspond à un gain en cytotoxicité obtenu à l'aide des conjugués spécifiques. Ainsi le ciblage de cellules tumorales par ces immunoconjugués réduit considérablement les concentrations efficaces du couple oxydase-peroxydase pouvant procurer une cytotoxicité spécifique. La figure 6 montre le gain en cytotoxicité spécifique et comporte en abscisse la concentration en g-GO ($\mu$g/ml) et en ordonnée le pourcentage d'absorbance.

La composition selon l'invention, de par la présence de deux immunotoxines entraîne une synergie de la toxicité spécifique, tout en neutralisant la toxicité non spécifique.

**Exemple 7 : Absence de toxicité des immuno-toxines de l'invention vis-à-vis des cellules souches de la moelle osseuse.**

Lors de l'application de la composition conforme à l'invention à l'épuration de la moelle osseuse de cellules cibles appropriées, il est nécessaire de s'assurer en particulier de l'absence de toxicité des immunotoxines vis-à-vis des cellules souches.

Une suspension de cellules de moelle osseuse est préincubée dans un sérum de lapin normal (NRS) dilué au 1/15e puis le ou les conjugués sont ajoutés comme indiqué ci-dessus. Les cellules sont incubées 20 min (exp. 1, 3, 4) ou 3 heures (exp. 2) dans un milieu CM, lavées et mises dans un milieu de clonage contenant de la carboxyméthylcellulose. Le nombre de colonies représentant des cellules souches hémato-poïétiques est compté 14 jours plus tard.

## **TABLEAU II**

| Traitement (μg/ml) | nombre de colonies récupérées /$10^5$ cellules nucléées de moelle osseuse |
|---|---|
| **1ère expérience** <br><br> - | <br><br> 244 ± 43 |
| g-GO + g-LPO <br> (0,05)  (0,3) | <br> 240 ± 28 |
| g-GO + g-LPO <br> (1)      (0,3) | <br> 313 ± 6 |
| g-GO + g-LPO <br> (1)      (2,5) | <br> 336 ± 26 |
| **2ème Expérience** <br><br> - | <br><br> 317 ± 24 |
| g-GO seule <br> (30) | <br> < 4 |
| g-LPO seule <br> (30) | <br> 225 ± 5 |
| g-GO + g-LPO <br> (10)      (30) | <br> 263 ± 9 |

| Traitement<br>(µg/ml) | nombre de colonies récupé-rées/$10^5$ cellules nucléées de moelle osseuse |
|---|---|
| **3ème expérience**<br><br>—<br><br>g-GO seule<br>(25) | < 4 |
| g-GO + g-LPO<br>(25)      (1) | < 4 |
| g-GO + g-LPO<br>(25)      (10) | 233 ± 2 |
| (25)      (75) | 267 ± 8 |
| **4ème expérience**<br>'<br>— | 312 ± 30 |
| GO seule<br>(25) | < 4 |
| GO + LPO<br>(500)  (300) | 244 |

Le Tableau II montre que les immunotoxines g-GO et g-LPO associées ne sont pas toxiques pour les cellules formant colonies de la moelle osseuse même à la dose cytotoxique optimale (1ère expérience).

Cependant l'incubation de cellules de la moelle dans 30 µg/ml de g-GO seule est très toxique pour les cellules et entraîne la destruction de tous les précurseurs (2ème expérience).

Dans une étude sur la relation dose-réponse de ce phénomène de protection observé, on a remarqué que 10 µg/ml de g-LPO protège partiellement les cellules souches et 75 µg/ml de g-LPO protège jusqu'à 76 % des cellules souches (3ème expérience). Ainsi, le conjugué g-LPO possède la propriété de neutraliser l'effet toxique non spécifique du conjugué g-GO.

La composition conforme à l'invention en associant une nitrosourée aux deux immunotoxines telles que définies, permet de réduire énormément les doses employées et de ce fait de telles compositions sont moins létales pour les cellules de l'environnement.

**Exemple 8 : Exemple de formulation.**

Une formulation de l'invention est préparée comme suit :

| | |
|---|---|
| DLC48-GO | 5 mg |
| DLC48-LPO | 5 mg |
| BCNU | 5 mg |
| SAH | 100 mg |
| Tampon acétate | 100 mM |
| Ethanol | 0,09 % |
| eau p.p.i. | 1 ml |

15

Lesdits composés sont mélangés, filtrés stérilement et conditionnés.

Une formulation lyophilisée peut être ensuite obtenue.

Ainsi que cela ressort de ce qui précède, l'invention ne se limite nullement à ceux de ses modes de mise en oeuvre, de réalisation et d'application qui viennent d'être décrits de fa'on plus explicité ; elle en embrasse au contraire toutes les variantes qui peuvent venir à l'esprit du technicien en la matière, sans s'écarter du cadre, ni de la portée, de la présente invention.

## Revendications

1°) Composition à propriétés cytolytiques spécifiques vis-à-vis de cellules cibles appropriées présentant au moins un antigène de surface, caractérisée en ce qu'elle comprend en combinaison :
- un inhibiteur de la glutathion réductase ;
- au moins une immunotoxine comprenant une enzyme conjuguée à un ligand spécifique d'un antigène de surface des cellules cibles.

2°) Composition selon la revendication 1, caractérisée en ce qu'elle comprend en combinaison :
- un inhibiteur de la glutathion réductase ;
- une première immunotoxine comprenant une enzyme $E_1$ productrice d'oxygène réactif conjuguée à un ligand A spécifique d'un antigène de surface desdites cellules cibles (immunotoxine $E_1$-A) ; et
- une deuxième immunotoxine comprenant une enzyme $E_2$, productrice de dérivés halogénés en présence d'un halogénure et de l'oxygène réactif produit en présence de l'enzyme $E_1$, conjuguée à un ligand B capable de se lier spécifiquement à un antigène de surface desdites cellules cibles (immunotoxine $E_2$-B).

3°) Composition selon l'une quelconque des revendications 1 et 2, caractérisée en ce que l'inhibiteur de la glutathion réductase est une nitrosourée.

4°) Composition selon la revendication 3, caractérisée en ce que la nitrosourée est avantageusement la 1-3 bis (2-chloroéthyl)-1-nitrosourée, également dénommée carmustine ou BCNU.

5°) Composition selon l'une quelconque des revendications 1 et 2, caractérisée en ce que l'enzyme de l'immunotoxine selon la revendication 1 ou de la première immunotoxine selon la revendication 2 est choisie dans le groupe qui comprend la glucose oxydase, la NADPH oxydase, la xanthine oxydase, la cholestérol oxydase, la choline oxydase, l'acide lactique oxydase et la pyruvate oxydase.

6°) Composition selon l'une quelconque des revendications 2 à 5, caractérisée en ce que l'enzyme $E_2$ est choisie dans le groupe qui comprend la myéloperoxydase, la lactoperoxydase, l'éosinophile peroxydase et la peroxydase salivaire.

7°) Composition selon l'une quelconque des revendications 1 à 6, caractérisée en ce que le ligand conjugué à une enzyme pour constituer une immunotoxine est choisi dans le groupe qui comprend les antisérums, les anticorps polyclonaux, les anticorps monoclonaux et les fragments d'anticorps, capables de se lier spécifiquement à un antigène de surface de la cellule cible.

8°) Composition selon l'une quelconque des revendications 2 à 7, caractérisée en ce que le ligand A et le ligand B sont identiques ou différents.

9°) Composition selon l'une quelconque des revendications 7 et 8, caractérisée en ce que lesdits ligands A et/ou B sont avantageusement choisis parmi les ligands des récepteurs de l'antigène cellulaire T et les ligands de l'immunoglobuline de surface.

10°) Composition selon l'une quelconque des revendications 1 à 9, caractérisée en ce que les cellules cibles appropriées sont choisies dans le groupe qui comprend les cellules tumorales, les bactéries, les champignons, les protozoaires, les cellules autoimmunes, les cellules inflammatoires, les cellules infectées par un virus, les levures, les virus, les parasites.

11°) Composition selon l'une quelconque des revendications 1 à 10, caractérisée en ce qu'elle contient en outre, une autre immunotoxine qui comprend une catalase conjuguée à un ligand C approprié, capable de se lier aux antigènes de surface de cellules présentes dans le même milieu que les cellules cibles (immunotoxine catalase-C).

12°) Composition selon l'une quelconque des revendications 1 à 11, caractérisée en ce qu'elle est associée à un véhicule propre à permettre son administration de préférence par voie parentérale et avantageusement en IV ou en perfusion.

13°) Procédé d'épuration *ex vivo* de la moelle osseuse de cellules cibles indésirables présentant au moins un antigène de surface, caractérisé en ce qu'il comprend les étapes suivantes :
(a) aspiration des cellules de la moelle osseuse,
(b) mise en contact desdites cellules avec une composition selon l'une quelconque des revendications 1 à 11, pendant un temps approprié.

14°) Procédé d'épuration de la moelle osseuse *ex vivo* de cellules cibles indésirables présentant au moins un antigène de surface, caractérisé en ce qu'il comprend successivement les étapes suivantes :

(a) aspiration des cellules de la moelle osseuse,

(b) mise en contact desdites cellules avec une quantité efficace d'inhibiteur de la glutathion réductase puis

(c) mise en contact desdites cellules avec une quantité efficace d'une immunotoxine constituée par un conjugué d'une enzyme avec un ligand capable de se lier spécifiquement à un antigène de surface spécifique des cellules cibles.

15°) Procédé d'épuration *ex vivo* de la moelle osseuse selon la revendication 14, caractérisée en ce qu'il comprend successivement les étapes suivantes :

(a) aspiration des cellules de la moelle osseuse,

(b) mise en contact desdites cellules avec une quantité efficace d'inhibiteur de la glutathion réductase puis

(c) mise en contact desdites cellules avec une quantité efficace d'une première immunotoxine constituée par un conjugué d'une enzyme $E_1$ générant de l'oxygène réactif avec un ligand A capable de se lier spécifiquement à un antigène de surface spécifique des cellules cibles, associée à une deuxième immunotoxine $E_2$-B, comprenant une enzyme $E_2$ productrice de dérivés halogénés en présence d'un halogénure et de l'oxygène réactif produit en présence de l'enzyme $E_1$, conjuguée à un ligand B capable de se lier spécifiquement à un antigène de surface desdites cellules cibles.

16°) Procédé d'épuration selon l'une quelconque des revendications 14 et 15, caractérisé en ce qu'il comprend en outre

(d) la mise en contact desdites cellules avec une autre immunotoxine catalase-C, comprenant un conjugué d'une catalase avec un un ligand C approprié, capable de se lier aux antigènes de surface de cellules présentes dans le même milieu que les cellules cibles.

17°) Procédé selon l'une quelconque des revendications 13 à 16, caractérisé en ce que l'inhibiteur de la gluthation réductase est une nitrosourée.

18°) Procédé selon la revendication 17, caractérisé en ce que la nitrosourée est avantageusement la 1-3 bis (2-chloroéthyl)-1-nitrosourée, également dénommée carmustine ou BCNU.

19°) Procédé selon l'une quelconque des revendications 13 à 18, caractérisé en ce que l'enzyme qui entre dans le conjugué qui forme l'immunotoxine ou la première immunotoxine est choisie dans le groupe qui comprend la glucose oxydase, la NADPH oxydase, la xanthine oxydase, la cholestérol oxydase, la choline oxydase, l'acide lactique oxydase et la pyruvate oxydase.

20°) Procédé selon l'une quelconque des revendications 15 à 19, caractérisé en ce que l'enzyme $E_2$ est choisie dans le groupe qui comprend la myéloperoxydase, la lactoperoxydase, l'éosinophile peroxydase et la peroxydase salivaire.

21°) Procédé selon l'une quelconque des revendications 14 à 20, caractérisé en ce que le ligand conjugué à l'enzyme selon la revendication 14 et/ou à chacune des enzymes selon la revendication 15 est choisi dans le groupe qui comprend les antisérums, les anticorps, les fragments d'anticorps capables de se lier spécifiquement à un antigène de surface de la cellule cible.

22°) Procédé selon l'une quelconque des revendications 13 à 21, caractérisé en ce que les cellules cibles à détruire sont des thymocytes et l'antigène de surface est une protéine caractéristique des thymocytes.

23°) Procédé selon la revendication 22, caractérisé en ce que ledit antigène de surface est choisi dans le groupe qui comprend l'antigène $T_4$, l'antigène $T_8$ et l'antigène Thyl.

24°) Procédé selon l'une quelconque des revendications 13 à 23, caractérisé en ce que les cellules cibles à détruire sont des cellules B de lymphomes.

17

FIG.1a

FIG.1b

FIG.1c

FIG.2

FIG.3a

EP 0 396 434 A1

FIG.3b

EP 0 396 434 A1

FIG.4a

FIG.4b

EP 0 396 434 A1

FIG.5

FIG.6

Office européen
des brevets

# RAPPORT DE RECHERCHE EUROPEENNE

Numero de la demande

EP 90 40 0486

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | BIOLOGICAL ABSTRACTS, vol. 79, no. 1, 1985, page AB-613, résumé no. 5581, Philadelphia, PA, US; F. DALLEGRI et al.: "Antibody-dependent killing of tumor cells by polymorphonuclear leukocytes: Involvement of oxidative and monoxidative mechanisms", & J. NATL. CANCER INST. 73(2): 331-340. 1984 * Résumé * | 1,3,4, 10 | A 61 K 39/395 A 61 K 47/00 |
| A | WO-A-8 703 205 (CORAL SOCIEDADE BRASILEIRA DE PESQUISAS E DESENVOLVIMENTO) * Revendications 1-7; page 2, lignes 29-34 * | 1,5,7, 10 | |

DOMAINES TECHNIQUES
RECHERCHES (Int. Cl.5)

A 61 K

Le présent rapport a été établi pour toutes les revendications

| Lieu de la recherche | Date d'achèvement de la recherche | Examinateur |
|---|---|---|
| LA HAYE | 11-07-1990 | PEETERS J.C. |

EPO FORM 1503 03.82 (P0402)